# EUROPEAN PATENT SPECIFICATION

(11) **EP 3 116 634 B1**
(45) Date of publication and mention of the grant of the patent: **06.06.2018**
(21) Application number: 15709177.8
(22) Date of filing: 11.03.2015
(51) Int. Cl.: B01D 69/08, C02F 3/12

(54) **AN AERATED BIOFILM REACTOR WITH HOLLOW FIBRE MEMBRANES**
BELÜFTETER BIOFILMREAKTOR MIT HOHLFASERMEMBRANEN
MEMBRANE DE RÉACTEUR À FIBRES CREUSES AVEC BIOFILM AÉRÉ

(30) Priority: 11.03.2014 GB 201404274
(43) Date of publication of application: 18.01.2017
(73) Proprietor: University College Dublin National University Of Ireland, Dublin, Dublin 4 (IE)
(72) Inventor: CASEY, Eoin, Clonskeagh, Dublin 14 (IE); SYRON, Eoin, Bray, County Wicklow (IE)
(74) Representative: FRKelly
(86) International application number: PCT/EP2015/055047
(87) International publication number: WO 2015/135977

(56) References cited:
- WO-A1-00/01472
- GB-A- 2 033 829
- JP-A- 2001 201 121
- US-A1- 2003 140 790
- US-A1- 2006 096 918
- US-A1- 2007 131 605
- US-A1- 2012 125 850

## Description

### Field of the invention

The present invention is concerned with a hollow fibre membrane for use in a Membrane Supported Biofilm Reactor (MSBR), or one embodiment of this reactor generally referred to as a membrane aerated biofilm reactor (MABR), and to a reactor utilising an array of such hollow fibre membranes, in particular for the large scale treatment of effluent such as municipal wastewater or the like. It will however be appreciated that the hollow fibre membrane of the invention may be used with any reactor which utilises one or more membranes to supply gas direclty to a biofilm.

### Background of the invention

In an MSBR, the biofilm is naturally immobilized on a gas permeable membrane. Oxygen or other gas diffuses through the membrane into the biofilm where oxidation of pollutants or biological reaction with other bio-available compounds, supplied at the biofilm-liquid interface, takes place. The gas supply rate is controlled by the intra-membrane gas partial pressure (a process parameter) and membrane surface area (a design parameter). The MABR concept is originally described in US. Patent No. 4,181,604, but successful commercialization has not materialized, primarily due to the difficulty in controlling the amount of biofilm in the reactor. In particular, excessive biofilm formation is known to cause clogging/channelling in the bioreactor, particularly in hollow fibre type systems, the dominant membrane configuration.

Biofilms, which comprise a community of microorganisms attached to a surface, have long been exploited for wastewater treatment. Natural immobilization of the microbial community on inert supports allows excellent biomass retention and accumulation without the need for solid-separation devices. In the context of wastewater treatment, the ability of biofilm based processes to completely uncouple solids retention time (SRT) from hydraulic retention time (HRT) is especially useful for slow-growing organisms which would otherwise be washed out of the system, nitrifying biofilms being a case in point. Established biofilm processes, such as the trickling filter became popular in the 20^{th} century because they offered simple, reliable and stable operation. Innovation in wastewater treatment technology is driven largely by the need to meet increasingly stringent regulatory standards and by the need to reduce the capital and operating costs of treatment processes. In recent years, these drivers have prompted the emergence of improved biofilm processes such as the Biological Aerated Filter (BAF) and the Moving Bed Biofilm Reactor (MBBR). One of the key advantages of biofilm-based processes is the potentially high volumetric reaction rate that can be attained due the high specific biomass concentration. Unfortunately, this advantage is rarely exploited in full-scale processes as a result of oxygen transfer limitations into thick biofilms. Biofilms in wastewater treatment systems are frequently thicker than the penetration depth of oxygen, typically 50µm to 150µm and, under high carbon-loading rates, the process becomes oxygen transfer rate limited. This problem, combined with the difficulty in controlling biofilm thickness has resulted in the application of biofilm technology predominantly for low-rate processes. Innovative technologies to overcome this problem are mainly based on methods that increase the specific surface area (particle based biofilm technologies), or on methods for increasing the oxidation capacity and efficiency, such as the membrane-aerated biofilm reactor (MABR).

The incorporation of membranes in wastewater treatment reactors can be traced back several decades when Schaffer et al (1960) reported the use of plastic films of unspecified material for oxygenation of a wastewater. Visible biological growth was observed on the polymer and it was reported that this had no observable effect on the oxygen transfer rate. It was not until 1978 when Yeh and Jenkins (1978) reported results of experiments with Teflon tubes in synthetic wastewater, that the potential of the membrane for oxygenation was recognized. This work was inspired by the emergence of hollow-fibre oxygenation systems for cell and tissue culture in the early 1970s. By 1980 the first patent was issued for a hollow fibre wastewater treatment reactor in which the biological oxidation takes place on the surface of micro-porous membranes. However, commercial exploitation of the technology has not yet emerged and until the present time there have been very limited trials of the technology beyond laboratory scale.

The MABR has several advantages over conventional biofilm technologies;
1. Comparatively high volumetric carbon oxygen demand (COD) removal rates are achievable if pure oxygen is fully exploited and if biofilm thickness-control measures are in place.
2. Bubbleless aeration offers the potential for significantly higher oxygen utilization efficiencies with consequent energy savings. In addition, reduced air stripping during the bio-treatment of volatile organic compounds is possible.
3. Simultaneous nitrification, denitrification and COD removal can be achieved at comparatively higher rates due to the unique microbial population stratification.
4. Specialist degrading microorganisms, such as ammonia oxidizing bacteria, tend to be preferentially located adjacent to the biofilm-membrane interface thereby enhancing their retention by protection from biofilm erosion.

Over a hundred research articles concerning both fundamental and applied aspects of the MABR have been published for a range of wastewater treatment application areas and the number of publications has surged dramatically in the past couple of years. The increased interest in the MABR has arisen perhaps due to a realization that it is a technology that can both achieve process intensification in wastewater treatment as well as offering the potential for significant energy cost savings.

There are a number of patents relating to MABR technology, however none of these incorporate effective biofilm control technology. EP2361367 aims to tackle the issue of biofilm control by providing the basis for determining when it is necessary to instigate the biofilm control.

To ensure the MABR can compete in the Waste Water Treatment marketplace there is a critical need to ensure that the oxygenation membranes have high oxygen permeability, are robust, cost effective and suitable for the immobilisation of biofilm. If the MABR is to achieve the potential indicated by laboratory scale trials, several technical challenges need to be overcome. The primary obstacle to full scale implementation has been the problem of excess biomass control which can lead to significant performance deterioration. In light of the method disclosed in EP2361367 to determine when biofilm control takes palace, it becomes necessary to prevent complete biofilm removal during the biofilm control procedure.

This is the paradox of the MABR, in that from a bio-catalytic point of view the more biofilm the better the reactor performs, however above a certain limit the accumulation of biofilm can cause severe problems with liquid flow distribution. The ideal MABR will operate in a cyclical manner with biofilm accumulation, partial removal and re-growth. In order to maintain the biofilm in the optimum range a mechanism to prevent complete biofilm detachment during the control operation is required. Many of the laboratory scale studies reported to-date in the literature were operated with low membrane packing densities and thus, the problem of biomass control was not prioritized. In assessing the prospect of the technology it is necessary to carefully examine the results of prior studies where modules were trialled using membrane packing densities high enough to be realistic for commercial application of MABR technology. Invariably these studies (Semmens et al, 2003, Semmens, 2005) have shown that significant clogging of the membrane module occurs, usually after several weeks or months of operation. This problem of excess biomass formation is concomitant with deterioration in the performance of the reactor in meeting its pollutant removal efficiencies.

U.S. Patent No. 4,181,604 (issued on Jan. 1, 1980), describes a module having several loops of hollow fibre porous membranes connected at both ends to a pipe at the bottom of a tank containing wastewater. The pipe carries oxygen to the lumens of the membranes and oxygen diffuses through the membrane pores to an aerobic biofilm growing on the outer surface of the membranes. In U.S. Patent No. 4,746,435 the same apparatus is used but the amount of oxygen containing gas is controlled to produce a biofilm having aerobic zones and anaerobic zones. U.S. Patent No. 6,558,549 describes an apparatus for treatment of wastewater where a biofilm is cultivated on the surface of non-rigid (sheet like) planar gas transfer membranes immersed in the wastewater tank in the vertical direction. The invention is an immersion type membrane system possibly for use in wastewater retrofit applications. There is however no effective means of biofilm thickness control. An air bubble scouring method is unlikely to be effective, and may remove all of the biofilm thereby impinging process performance.

US Patent No. 5,403,479 describes an in situ cleaning system for fouled membranes. The membrane is cleaned by a cleaning fluid containing a biocide. US Patent 5,941,257 describes a method for two-phase flow hydrodynamic cleaning for piping systems. US Patent No. 7,367,346 describes a method for cleaning hollow tubing and fibres. These three patents are applied for the cleaning hemodialyzers used for dialysis and hollow fibre modules used in water treatment and separations. They are not applicable to systems where the material to be cleaned is acting as a biocatalyst and do not have any form of process sensing linked to the cleaning method.

US2006096918 disclosed biofilters and methods for using them. The biofilters described include a network of hollow fiber membranes upon which a biofilm is caused to develop, filling the interstices between the membranes.

US2012125850 discloses a porous hollow fiber membrane that is suitable for treatment of liquid containing an inorganic substance and/or an organic substance, is obtained at a low cost performance, and has high water permeability performance, fretting resistance, and drying resistance. A deformed porous hollow fiber membrane is composed of a thermoplastic resin and includes a continuous asperity provided on the periphery in the longitudinal direction of the membrane, in which the periphery of the hollow fiber membrane in the circumferential direction includes continuous projected and depressed parts.

US2003140790 discloses porous hollow polymer fiber membranes having convoluted inside and/or outside surfaces, as well as filter devices comprising a plurality of the hollow fiber membranes, the devices preferably being arranged to direct fluid flow from the inside surface of the membranes to the outside surface, methods of making the membranes, and methods of using the filter devices.

GB2033829 discloses a hollow fibre of cellulose regenerated from cuprammonium solutions for use as a dialysis membrane has a continuous cavity and a surface which is formed with periodic changes in shape in the longitudinal and/or circumferential direction thereof.

WO0001472 discloses a hollow fiber membrane for increasing gas exchange between two fluids wherein one of the fluids flows through the membrane and the second fluid flows around the membrane and the membrane has an exterior surface and an interior surface and a plurality of protuberances for disrupting the boundary layer of one of the two fluids.

The present invention seeks to provide an improved hollow fibre membrane for use with membrane aerated biofilm reactors.

### Summary of the invention

According to a first aspect of the present invention there is provided an aerated biofilm reactor fibre membrane comprising a substantially cylindrical sidewall defining an internal lumen from which gas can permeate through the sidewall; characterised in that at least a part of an outer surface of the fibre membrane is engineered to define at least one biofilm retaining region within its cross-section.

Preferably, the outer surface of the fibre membrane defines an array of the engineered biofilm retaining regions.

Preferably, the engineered biofilm retaining region of the outer surface comprises one or more concave regions.

Preferably, the engineered biofilm retaining region of the outer surface comprises one ore more substantially radially extending protrusions.

Preferably, the engineered biofilm retaining region of the outer surface comprises one ore more substantially longitudinally extending corrugations.

Preferably, the outer surface of the fibre membrane is multilateral.

Preferably, an inner surface of the fibre membrane, which defines the lumen, is shaped to optimise gas transfer through the sidewall.

Preferably, the fibre membrane is formed as a polymer extrusion.

Preferably, the fibre membrane comprises an open end through which gas may be supplied to the lumen.

Preferably, the fibre membrane has an external diameter in the range of between 0.2mm to 5mm, more preferably between 0.35mm and 0.9mm, and most preferably 0.5mm.

Preferably, the fibre membrane comprises a gas permeable polymer.

Preferably, the fibre membrane comprises polydimethyl siloxane (PDMS).

According to a second aspect of the present invention there is provided a membrane aerated biofilm reactor comprising a plurality of hollow fibre membranes according to the first aspect of the invention.

Preferably, the reactor comprises means for supplying a gas to the lumen of the fibre membranes.

Preferably, at least an open end of each fibre membrane is captured in an anchor.

Preferably, the fibre membranes are arranged in groups.

### Brief description of the drawings

Figure 1 illustrates a cross section of an conventional prior art hollow fibre for use in a membrane aerated biofilm reactor;
Figure 2 illustrates a cross section of an aerated biofilm reactor membrane fibre according to a preferred embodiment of the present invention;
Figure 3 illustrates a cross section of an alternative aerated biofilm reactor membrane fibre according to a preferred embodiment of the present invention;
Figure 4 illustrates a cross section of another alternative aerated biofilm reactor membrane fibre according to a preferred embodiment of the present invention;
Figure 5 illustrates a cross section of another alternative aerated biofilm reactor membrane fibre according to a preferred embodiment of the present invention
Figure 6 illustrates a cross section of another alternative aerated biofilm reactor membrane fibre according to a preferred embodiment of the present invention
Figure 7 illustrates a cross section of another alternative aerated biofilm reactor membrane fibre according to a preferred embodiment of the present invention; and
Figure 8 illustrates a cross section of an aerated biofilm reactor membrane fibre not in accordance with the present invention.

### Detailed description of the drawings

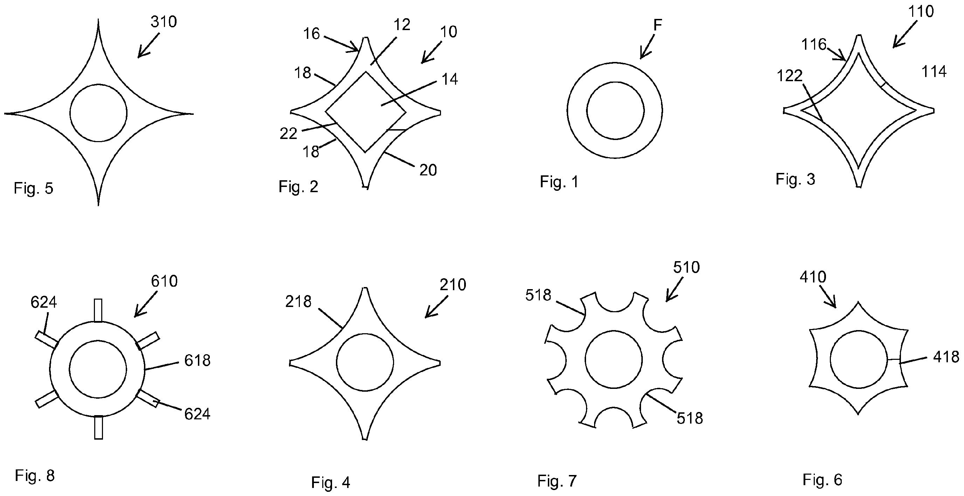

Referring now to Figure 1 there is shown a cross-section of a conventional prior art hollow fibre F for use in a membrane aerated biofilm reactor (not shown). The hollow fibre F is substantially cylindrical in cross-section and defines an interior lumen L through which gas such as oxygen, air, oxygen enriched air, hydrogen or any other suitable gas, may be supplied, and which then permeates through the sidewall of the hollow fibre F in order to, in use, oxygenate a biofilm colonising the outer surface of the hollow fibre F. It can be seen that the outer surface of the hollow fibre F is a substantially smooth and continuous surface.

Turning then to Figure 2 there is illustrated a cross-section of a fibre membrane for use in a membrane aerated biofilm reactor (not shown), the fibre membrane being generally indicated as 10. The fibre membrane 10 comprises a substantially cylindrical side wall 12 which is annular in form, and thus defines an interior lumen 14 which extends longitudinally of the fibre membrane 10. In use a gas such as oxygen or the like is pumped into the lumen 14 and, by providing the sidewall 12 as a gas permeable material, the gas can permeate through the sidewall 12 to be supplied to a biofilm (not shown) colonizing an outer surface 16 of the fibre membrane 10. Unlike prior art hollow fibres, the fibre membrane 10 of the present invention defines one or more, and preferably a plurality of, engineered biofilm retaining regions 18 which, as described in detail hereinafter, act to retain a quantity of biofilm therein, in particular when the fibre membrane 10 is subjected to a high sheer biofilm control event, such as experienced during a reactor cleaning cycle, for removing excess biofilm in order to prevent clogging of the reactor. As a result, once such an event has been completed, the biofilm held in the retaining regions 18 ensure expedient regrowth of the biofilm to full operational levels, thus significantly reducing the lead time between such a cleaning event and a return to full operation of the reactor. Conventionally this would be a significantly longer period in order to facilitate reseeding of the biofilm and regrowth on the outer surface of the fibre to an operational level.

Unlike prior art fibres, in the Figure 2 embodiment the outer surface 16 is multilateral, and includes four concave sides 20, each of which defines a single biofilm retaining region 18. It can be seen that the inner surface 22 of the sidewall 12 is also multilateral, corresponding in number of sides to that of the outer surface 16, although each of the sides are relatively flat as opposed to the concave sides 20 of the outer surface 16. It will however be appreciated that the shape of both the outer surface 16 and inner surface 22 may be varied as required. For example it may be preferable that the outer surface 16 and inner surface 22 are substantially parallel in order to provide the side wall 12 with a substantially uniform thickness, thereby ensuring an equal transfer of gas at all points around the side wall 12, in order to establish an equal growth rate of biofilm about the outer surface 16. Equally however it will understood that it may be desirable to encourage regions of increased or decreased biofilm thickness on the outer surface 16, by suitably altering the gas permeability of that region of the sidewall 12, for example by varying the thickness of the sidewall 12 at localised regions. The fibre membrane 12 preferably has a external diameter in the range of between 0.2mm to 5mm, more preferably between 0.35mm and 0.9mm, and most preferably 0.5mm, which diameter is measured at the radially outmost extremity of the fibre membrane 12.

The fibre membrane 12 is preferably produced by extruding a polymer through a suitably shaped die (not shown) to provide the desired external and internal profiles to the fibre membrane 10. It will however be immediately understood that any other suitable method of manufacturing the fibre membrane 10 may be employed, and the material or combination of materials selected to form the fibre membrane 10 may be varied. The fibre membrane 12 is preferably comprised of silicone (polydimethyl siloxane (PDMS)) Or a modified version of PDMS, although other suitable materials may be employed.

Referring to Figures 3 to 8 there are illustrated alternative embodiments of a fibre membrane according to the present invention and for use in a MABR, each variant providing an alternative sidewall profile, as dictated by the shape of an outer surface and/or an inner surface of the respective fibre membrane.

In particular, referring to Figure 3 there is illustrated a fibre membrane 110 similar in cross-section to the fibre membrane 10 of the first embodiment. However, an outer surface 116 and an inner surface 122 are substantially parallel with one another, thus providing the side wall 112 with a substantially uniform thickness.

Figure 4 illustrates a fibre membrane 210 having a multilateral outer surface 216 and a substantially circular inner surface 222 defining an interior lumen 214.

The cross-section illustrated in Figure 5 is similar to that shown in Figure 4, illustrating a fibre membrane 310 having a multilateral outer surface 316 comprising four concave sides 320 which each define a biofilm retaining region 318, separated from one another by a more pronounced or sharp apex. An inner surface 322 is substantially circular in cross-section.

The cross-section of illustrated in Figure 6 illustrates a fibre membrane 410 which is again multilateral in form, defining six concave sides 420 and a substantially circular inner surface 422.

Turning to Figure 7, there is illustrated a fibre membrane 510 having an outer surface 516 from which protrude a plurality of substantially radially extending projections 524 between adjacent pairs of which are thus defined a biofilm retaining region 518 in the form of a concave corrugation 520, each of which extends substantially longitudinally of the fibre membrane 510.

Figure 8 illustrates a fibre membrane 610 not in accordance with the invention and which comprises a side wall 612 having a substantially circular outer surface 616 and a substantially circular inner surface 622. However the side wall 612 is further provided with a circular array of substantially radially extending protrusions 624 between adjacent pairs of which are thus defined biofilm retaining regions 618 which extend substantially longitudinally of the fibre membrane 610.

In each of the above fibre membranes at least one, and preferably an array of, biofilm retaining regions are defined about an outer surface of the fibre membrane, such that during a high sheer event such as a biofilm purge in order to prevent clogging of a reactor, some level of biofilm is retained in the retaining regions on the outer surface of each fibres membrane, in order to facilitate a speedy regrowth of the biofilm following the high shear event, in order to allow the reactor to be fully operational in a reduced period of time.

## Claims

1. A membrane aerated biofilm reactor comprising a plurality of hollow fibre membranes (10; 110; 210; 310; 410; 510) each comprising a substantially cylindrical sidewall (12) defining an internal lumen (14) from which gas can permeate through the sidewall; means for supplying a gas to the lumen of one or more of the fibre membranes; **characterised in that** at least a part of an outer surface (16) of the fibre membrane is engineered to define at least one concave biofilm retaining region (18) within its cross-section.

2. A membrane aerated biofilm reactor according to claim 1 in which the outer surface of the fibre membrane defines an array of the engineered biofilm retaining regions.

3. A membrane aerated biofilm reactor according to claim 1 or 2 in which the engineered biofilm retaining region of the outer surface comprises one ore more substantially longitudinally extending corrugations.

4. A membrane aerated biofilm reactor according to claim 1 or 2 in which the outer surface of the fibre membrane is multilateral.

5. A membrane aerated biofilm reactor according to claim 1 or 2 in which the fibre membrane is formable by polymer extrusion.

6. A membrane aerated biofilm reactor according to claim 1 or 2 in which the fibre membrane comprises an open end opposed closed end and through which gas may be supplied to the lumen.

7. A membrane aerated biofilm reactor according to claim 1 or 2 in which the fibre membrane has an external diameter in the range of between 0.2mm to 5mm, more preferably between 0.35mm and 0.9mm, and most preferably 0.5mm.

8. A membrane aerated biofilm reactor according to claim 1 or 2 in which the fibre membrane comprises a gas permeable polymer.

9. A membrane aerated biofilm reactor according to claim 1 or 2 in which the fibre membrane comprises polydimethyl siloxane (PDMS).

10. A membrane aerated biofilm reactor according to any preceding claim in which at least an open end of each fibre membrane is captured in an anchor.

11. A membrane aerated biofilm reactor according to any preceding claim in which the fibre membranes are arranged in groups.

## Patentansprüche

1. Membran-belüfteter Biofilmreaktor, der Folgendes umfasst: eine Vielzahl von Hohlfasermembranen (10; 110; 210; 310; 410; 510), die jeweils eine im Wesentlichen zylindrische Seitenwand (12) umfassen, die einen inneren Hohlraum (14) definiert, aus dem Gas durch die Seitenwand dringen kann; Mittel für die Zuführung eines Gases zu dem Hohlraum von einer oder mehreren der Fasermembranen; **dadurch gekennzeichnet, dass** zumindest ein Teil einer äußeren Oberfläche (16) der Fasermembran konstruiert ist, um mindestens einen konkaven Biofilm-zurückhaltenden Bereich (18) innerhalb ihres Querschnitts zu definieren.

2. Membran-belüfteter Biofilmreaktor nach Anspruch 1, in dem die äußere Oberfläche der Fasermembran eine Anordnung der konstruierten Biofilm-zurückhaltenden Bereiche definiert.

3. Membran-belüfteter Biofilmreaktor nach Anspruch 1 oder 2, in dem der konstruierte Biofilm-zurückhaltende Bereich der äußeren Oberfläche eine oder mehrere im Wesentlichen sich längs erstreckende Wellungen umfasst.

4. Membran-belüfteter Biofilmreaktor nach Anspruch 1 oder 2, in dem die äußere Oberfläche der Fasermembran multilateral ist.

5. Membran-belüfteter Biofilmreaktor nach Anspruch 1 oder 2, in dem die Fasermembran durch Polymerextrusion formbar ist.

6. Membran-belüfteter Biofilmreaktor nach Anspruch 1 oder 2, in dem die Fasermembran ein offenes Ende, das einem geschlossenen Ende gegenüberliegt, umfasst und durch das Gas zu dem Hohlraum zugeführt werden kann.

7. Membran-belüfteter Biofilmreaktor nach Anspruch 1 oder 2, in dem die Fasermembran einen Außendurchmesser im Bereich von zwischen 0,2 mm bis 5 mm, bevorzugter zwischen 0,35 mm und 0,9 mm und am bevorzugtesten 0,5 mm aufweist.

8. Membran-belüfteter Biofilmreaktor nach Anspruch 1 oder 2, in dem die Fasermembran ein gasdurchlässiges Polymer umfasst.

9. Membran-belüfteter Biofilmreaktor nach Anspruch 1 oder 2, in dem die Fasermembran Polydimethylsiloxan (PDMS) umfasst.

10. Membran-belüfteter Biofilmreaktor nach einem vorstehenden Anspruch, in dem mindestens ein offenes Ende von jeder Fasermembran
in einer Verankerung festgehalten ist.

11. Membran-belüfteter Biofilmreaktor nach einem vorstehenden Anspruch, in dem die Fasermembranen in Gruppen angeordnet sind.

## Revendications

1. Réacteur à biofilm aéré sur membrane comprenant une pluralité de membranes de fibres creuses (10 ; 110 ; 210 ; 310 ; 410 ; 510) comprenant chacune une paroi latérale substantiellement cylindrique (12) définissant une lumière interne (14) depuis laquelle du gaz peut s'infiltrer à travers la paroi latérale ; un moyen pour amener un gaz vers la lumière d'une ou de plusieurs des membranes de fibres ; **caractérisé en ce qu'**au moins une partie d'une surface externe (16) de la membrane de fibres est conçue pour définir au moins une région de retenue de biofilm concave (18) au sein de sa section transversale.

2. Réacteur à biofilm aéré sur membrane selon la revendication 1 dans lequel la surface externe de la membrane de fibres définit un agencement des régions de retenue de biofilm mises au point.

3. Réacteur à biofilm aéré sur membrane selon la revendication 1 ou 2 dans lequel la région de retenue de biofilm mise au point de la surface externe comprend une ou plusieurs ondulations s'étendant substantiellement longitudinalement.

4. Réacteur à biofilm aéré sur membrane selon la revendication 1 ou 2 dans lequel la surface externe de la membrane de fibres est multilatérale.

5. Réacteur à biofilm aéré sur membrane selon la revendication 1 ou 2 dans lequel la membrane de fibres est capable d'être formée par l'extrusion d'un polymère.

6. Réacteur à biofilm aéré sur membrane selon la revendication 1 ou 2 dans lequel la membrane de fibres comprend une extrémité ouverte opposée à une extrémité fermée et par laquelle du gaz peut être amené vers la lumière.

7. Réacteur à biofilm aéré sur membrane selon la revendication 1 ou 2 dans lequel la membrane de fibres a un diamètre externe dans la plage de 0,2 mm à 5 mm, plus préférablement de 0,35 mm à 0,9 mm, et de manière plus préférée entre toutes de 0,5 mm.

8. Réacteur à biofilm aéré sur membrane selon la revendication 1 ou 2 dans lequel la membrane de fibres comprend un polymère perméable au gaz.

9. Réacteur à biofilm aéré sur membrane selon la revendication 1 ou 2 dans lequel la membrane de fibres comprend du polydiméthylsiloxane (PDMS).

10. Réacteur à biofilm aéré sur membrane selon l'une quelconque des revendications précédentes dans lequel au moins une extrémité ouverte de chaque membrane de fibres est capturée dans une ancre.

11. Réacteur à biofilm aéré sur membrane selon l'une quelconque des revendications précédentes dans lequel les membranes de fibres sont agencées en groupe.
